# FASCICULE DE BREVET EUROPEEN

(11) **EP 1 536 735 B1**
(45) Date de publication et mention de la délivrance du brevet: **22.11.2006**
(21) Numéro de dépôt: 03795041.7
(22) Date de dépôt: 11.09.2003
(51) Int. Cl.: A61B 17/70

(54) **ELEMENT DE LIASON POUR LA STABILISATION DYNAMIQUE D'UN SYSTEME DE FIXATION RACHIDIEN**
VERBINDUNGSELEMENT FÜR DIE DYNAMISCHE STABILISIERUNG EINES WIRBELSÄULENFIXIERSYSTEMS UND DIESES ENTHALTENDES WIRBELSÄULENFIXIERSYSTEM
LINKING ELEMENT FOR DYNAMICALLY STABILIZING A SPINAL FIXING SYSTEM AND SPINAL FIXING SYSTEM COMPRISING SAME

(30) Priorité: 11.09.2002 FR 0211251
(43) Date de publication de la demande: 08.06.2005
(73) Titulaire: Spinevision, 75012 Paris (FR)
(72) Inventeur: PETIT, Dominique, F-62180 Verton (FR)
(74) Mandataire: Breese, Pierre
(86) Numéro de dépôt international: PCT/FR2003/002695
(87) Numéro de publication internationale: WO 2004/024011

(56) Documents cités:
- EP-A- 0 516 567
- FR-A- 2 702 363
- FR-A- 2 717 370
- FR-A- 2 718 946
- US-A- 4 697 582
- US-A- 5 672 175

## Description

La présente invention se rapporte au domaine des systèmes rachidiens pour la liaison des vertèbres.

Il existe deux types de liaisons rachidiennes : les liaisons d'ostéosynthèse et les liaisons dynamiques.

Les liaisons d'ostéosynthèse rachidiennes ont pour but de figer les vertèbres liées dans une configuration particulière et de stabiliser celles-ci pendant la fusion osseuse afin de permettre une stabilisation fixe dans la situation figée.

Les liaisons dynamiques, au contraire, n'engendrent pas la fusion osseuse, mais cherchent à réduire les contraintes sur les facettes articulaires et sur les disques intervertébraux, en autorisant certains mouvements, tout en réalignant les vertèbres les unes par rapport aux autres si nécessaire.

Des systèmes de ce type sont montrés dans les documents US-A-4,697,582 et FR-A-2,717,370.

La présente invention se rapporte plus particulièrement à un élément de liaison et de stabilisation dynamique pour un système de fixation rachidien, destiné à lier au moins deux ensembles de connexion implantables l'un avec l'autre, autorisant certaines possibilités de mouvement de l'un par rapport à l'autre.

L'art antérieur connaît déjà des éléments de liaison pour la stabilisation dynamique.

Il est proposé, dans la demande de brevet européen EP669109, un dispositif de stabilisation de vertèbres dorsales avoisinantes comportant une bande qui est réalisée en matière synthétique élastique et qui présente une section transversale ronde, et au moins deux vis de pédicule. La bande peut être insérée à travers le perçage transversal de la tête de vis des vis et fixée par une vis de serrage transversalement au perçage, c'est-à-dire en direction de l'axe de vis.

Le dispositif comprend en outre un élément d'appui monté sur la bande. Cet élément d'appui forme un corps résistant à la pression pour transférer des forces de pression entre les deux têtes de vis. La section transversale de la bande s'applique de tous côtés dans des perçages adaptés de l'élément d'appui et de la tête de vis pour centrer l'élément d'appui et la tête de vis l'un relativement à l'autre.

En outre, la bande peut être précontrainte entre deux vis de pédicule avoisinantes par un prolongement de bande dépassant à l'extérieur des vis de pédicule pour pouvoir supporter mutuellement l'élément d'appui et la tête de vis sur une surface d'appui qui leur est commune, répartie autour de la bande.

Un inconvénient majeur de ce dispositif de stabilisation est que la bande doit être glissée dans le trou ménagé dans la tête des vis pédiculaires, ainsi qu'au travers du ou des élément(s) d'appui. Cela implique tout d'abord une difficulté lors de l'implantation, d'enfiler la bande au travers de ces éléments, les vis pédiculaires étant déjà fixées aux vertèbres. L'introduction de la bande n'est pas aisée ; mais cela implique surtout qu'il est impératif de choisir la longueur de l'élément d'appui avant la mise en place de la bande. Or il peut arriver que la distance effective entre les vis après mise en tension de la bande, ne soit pas exactement celle souhaitée, dans ce cas, le chirurgien n'a d'autre choix que de démonter l'ensemble élément d'appui, bande pour introduire un élément d'appui d'une longueur différente. En effet, ce système ne permet aucune liberté de compression distraction entre les vis après mise en place de l'ensemble des éléments. De plus la matière viscoélastique de l'élément d'appui se comprime lors de la mise en tension de la bande ce qui complique encore le choix de longueur de l'élément d'appui puisque celle-ci change lors de la mise en tension de la bande.

Un autre inconvénient majeur du dispositif de stabilisation de l'art antérieur est que l'élément de liaison occupe un volume important : de l'ordre de 12,5 millimètres. Dans certaines circonstances, il est alors difficile d'empêcher que l'élément de liaison ne rentre en contact avec des os et que ce contact provoque de grandes douleurs.

Un autre inconvénient majeur du dispositif de stabilisation de l'art antérieur est que l'élément de liaison n'effectue aucun rappel en torsion afin de s'opposer à des mouvements de pivotement des vertèbres autour des disques.

Un autre inconvénient majeur du dispositif de stabilisation de l'art antérieur est que l'élément de liaison ne peut pas être cintré pour permettre de l'adapter à la lordose naturelle du rachis lombaire.

Un autre inconvénient majeur du dispositif est l'impossibilité d'effectuer une reprise chirurgicale d'extension de montage, ou bien encore le remplacement de l'élément de liaison par une tige rigide pour obtenir une ostéosynthèse sans procéder à l'ablation complète du matériel au préalable.

Un autre inconvénient du dispositif est l'impossibilité de le combiner avec un système classique d'ostéosynthèse.

La présente invention entend remédier aux inconvénients de l'art antérieur en proposant un élément de liaison pour un système de fixation rachidien qui puisse être inséré dans un ensemble de connexion implantable sur la tête de cet ensemble, de manière traditionnelle, comme une tige de liaison pour ostéosynthèse, qui présente un diamètre réduit, qui réalise un rappel en torsion afin d'inciter un retour à une position d'équilibre, qui puisse être cintré, qui puisse être combiné avec un système d'ostéosynthèse, et encore qui puisse être facilement interchangé pour réaliser une ostéosynthèse.

Pour ce faire, la présente invention est du type décrit dans la revendication 1.

Ledit support est, de préférence, constitué d'une forme sensiblement tubulaire ou cylindrique.

Avantageusement, lesdites spires de la seconde tige forment un ressort hélicoïdal sensiblement coaxial avec ledit support.

Ladite tige présente, de préférence, un diamètre extérieur inférieur au diamètre intérieur desdites spires.

Pour réaliser une ostéosynthèse, l'élément de liaison comporte, de préférence, un élément rigidificateur droit ou cintré.

Cet élément rigidificateur est, de préférence, constitué d'une feuille de matériau présentant sensiblement en coupe transversale une forme de U.

La présente invention se rapporte également à un système de fixation rachidien comportant au moins deux ensembles de connexion implantables liés à l'aide d'au moins un élément de liaison selon l'invention.

Pour réaliser une ostéosynthèse, lorsque le système de fixation comporte un élément rigidificateur, cet élément est, de préférence, fixé au moins aux deux ensembles de connexion implantables.

Pour réaliser une ostéosynthèse, le système de fixation peut en outre comporter au moins un élément de liaison rigide.

Avantageusement, l'insertion de l'élément de liaison selon l'invention dans les moyens d'accueil d'un ensemble de connexion implantable de type connu est simple à réaliser. La mise en place de l'élément de liaison selon l'invention n'implique pas l'utilisation d'un implant spécifiquement conçu pour cela. La technique implantatoire est donc la même que pour la fusion.

Avantageusement également, la structure de l'élément de liaison selon l'invention permet d'obtenir les contraintes de résistance à la compression et à l'extension souhaitée, tout en ne nécessitant qu'un volume réduit. En outre, le diamètre extérieur de l'élément de liaison selon l'invention étant sensiblement le même que celui des éléments de liaison utilisés pour la fusion, il est possible de réaliser dans un même système de fixation une combinaison de liaisons transversales pour la fusion et de liaisons transversales pour la stabilisation dynamique.

Avantageusement également, la structure de l'élément de liaison selon l'invention permet également de réaliser un rappel en torsion afin de contraindre au retour vers une configuration d'équilibre déterminée.

Avantageusement également, la structure de l'élément de liaison selon l'invention permet de l'adapter à la configuration de l'implantation, en lui donnant la forme désirée, et notamment en le pré cintrant.

Avantageusement également, la structure de l'élément de liaison selon l'invention permet de réaliser la compression et la distraction directement sur les vis

On comprendra mieux l'invention à l'aide de la description, faite ci-après à titre purement explicatif, d'un mode de réalisation de l'invention, en référence aux figures annexées :
- la figure 1 illustre une vue de face d'une première variante de réalisation de l'élément de liaison selon l'invention, droit ;
- la figure 2 illustre une vue en coupe selon AA de la figure 1 ;
- la figure 3 illustre une vue de face d'une première variante de réalisation de l'élément de liaison selon l'invention, cintré ;
- la figure 4 illustre une vue de face d'une deuxième variante de réalisation de l'élément de liaison selon l'invention, droit ;
- la figure 5 illustre une vue en coupe selon AA de la figure 4 ;
- la figure 6 illustre une vue de face d'une deuxième variante de réalisation de l'élément de liaison selon l'invention, cintré ;
- la figure 7 illustre une vue de face d'une troisième variante de réalisation de l'élément de liaison selon l'invention, droit ;
- la figure 8 illustre une vue en coupe selon AA de la figure 7 ;
- la figure 9 illustre une vue de face d'une troisième variante de réalisation de l'élément de liaison selon l'invention, cintré ;
- la figure 10 illustre une vue en coupe éclatée d'une première variante d'un ensemble de connexion pour un élément de liaison selon l'invention ;
- la figure 11 illustre une vue de face, monté, de l'ensemble de connexion de la figure 10 ;
- la figure 12 illustre une vue de côté, des moyens d'accueil de l'ensemble de connexion de la figure 10 ;
- la figure 13 illustre une vue de face, des moyens d'accueil de l'ensemble de connexion de la figure 10 ;
- la figure 14 illustre une vue de face d'un système de fixation comportant deux ensembles de connexion selon la première variante et un élément de liaison selon l'invention droit ;
- la figure 15 illustre une vue de face d'un système de fixation rachidien comportant quatre ensembles de connexion selon la première variante et un élément de liaison selon l'invention droit, rigidifié sur une portion droite ;
- la figure 16 illustre une vue en perspective de la figure 15 ;
- la figure 17 illustre une vue de face d'un système de fixation comportant quatre ensembles de connexion selon la première variante et un élément de liaison selon l'invention cintré, rigidifié sur une portion droite ;
- la figure 18 illustre une vue de face d'un système de fixation comportant-quatre ensembles de connexion selon la première variante et un élément de liaison selon l'invention cintré, rigidifié sur une portion cintrée ;
- la figure 19 illustre une vue de dessus d'un système de fixation comportant douze ensembles de connexion selon la première variante, quatre éléments de liaison selon l'invention droits, deux éléments de liaison rigides et quatre dominos, pour lier six vertèbres entre elles ;
- la figure 20 illustre une vue en coupe d'une deuxième variante d'un ensemble de connexion pour un élément de liaison selon l'invention ;
- la figure 21 illustre une vue partielle en coupe d'une troisième variante d'un ensemble de connexion pour un élément de liaison selon l'invention ;
- la figure 22 illustre une vue en perspective de la figure 21 ; et
- la figure 23 illustre une vue de face d'un système de fixation comportant deux ensembles de connexion selon la deuxième variante, deux ensembles de connexion selon la troisième variante, deux éléments de liaison selon l'invention cintrés et un élément de liaison rigide.

L'élément de liaison (101, 201, 201') pour système de fixation rachidien (100, 200) selon l'invention est destiné à lier au moins deux ensembles de connexion (102, 202) implantables dans le rachis à l'aide de moyens d'ancrage osseux.

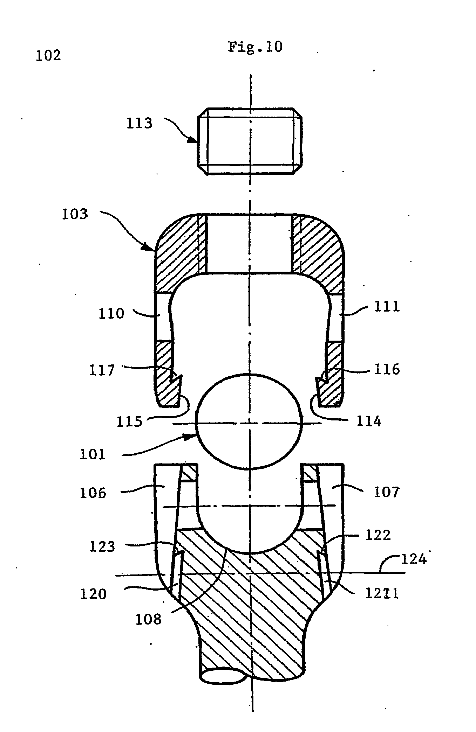

Un ensemble de connexion implantables (102) selon la première variante de mise en oeuvre de l'invention est un ensemble tel que celui connu de la demande internationale de brevet N° WO 01/01873 et dont la coopération avec l'élément de liaison selon la présente invention est décrite plus en détail ci-après, en référence aux figures 10 à 19.

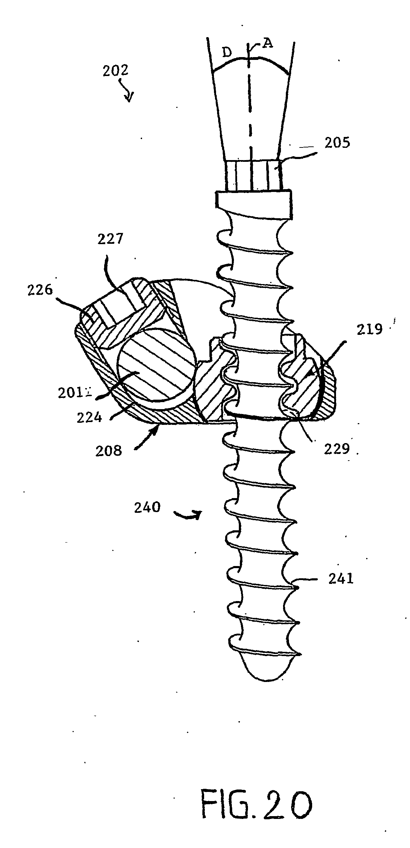

Un ensemble de connexion implantables (202) selon la deuxième variante de mise en oeuvre de l'invention est un ensemble tel que celui connu de la demande internationale de brevet N° WO 01/39677 et dont la coopération avec l'élément de liaison selon la présente invention est décrite plus en détail ci-après, en référence aux figures 20 à 23.

L'élément de liaison (101, 201, 201') est sensiblement tubulaire ou cylindrique et présente un axe longitudinal. Son diamètre extérieur est de l'ordre de 6 millimètres.

L'élément de liaison (101, 201, 201') selon l'invention est constitué, au moins partiellement :
- d'une part d'un ressort hélicoïdal (150, 250) présentant un axe (155, 255) et des spires (152, 252) et,
- d'autre part d'un support (160, 260) en matériau polymère présentant un axe (165, 265) sensiblement parallèle avec l'axe dudit ressort hélicoïdal (150, 250).

Lesdites spires sont au moins partiellement, noyées dans au moins un support (160, 260) en matériau polymère.

Le ressort hélicoïdal (150, 250) peut être réalisé en métal, ou en alliage métallique, ou encore dans tout type de matériau biocompatible permettant la réalisation d'un tel ressort. Il présente des spires (152, 252) présentant un diamètre extérieur (151, 251) et un diamètre intérieur (153, 253). Le diamètre de la tige de métal réalisant les spires est de l'ordre de 0,8 millimètres pour un matériau en alliage à base de titane.

Le support (160, 260) peut être réalisé, par exemple, en biomatériau élastique (polycarbonate, polyvinyl, alcool, etc.) Il est constitué d'une forme sensiblement tubulaire ou cylindrique et présente un diamètre extérieur (162, 262) et, éventuellement un diamètre intérieur (163, 263).

Le diamètre extérieur (162, 262) du support (160, 260) peut être supérieur au diamètre extérieur (151, 251) du ressort hélicoïdal (150, 250), et le support (160, 260) peut par conséquent inclure complètement le ressort hélicoïdal (150, 250), toutefois, ce diamètre extérieur (162, 262) ne peut être inférieur au diamètre intérieur (153, 253) du ressort hélicoïdal (150, 250). En d'autres termes, en coupe transversale, les spires (152, 252) sont toujours au moins partiellement incluses dans du matériau polymère du support (160, 260).

Il est également possible d'imaginer, bien que cela ne soit pas illustré, que le diamètre intérieur (163, 263) du support (160, 260) soit supérieur au diamètre intérieur (153, 253) du ressort hélicoïdal (150, 250).

Dans les éléments de liaison (101, 201, 201') illustrés, le ressort hélicoïdal (150, 250) et le support (160, 260) présentent une longueur sensiblement identique ; toutefois, il peut être imaginé qu'à une extrémité ou aux deux, le ressort soit plus court que le support, ou inversement.

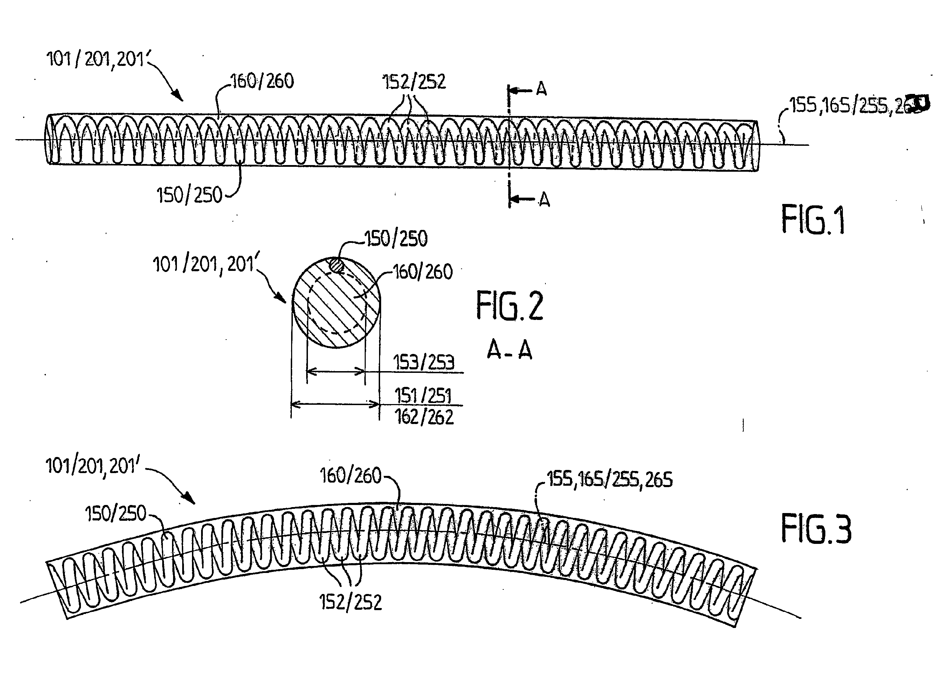

Les figures 1 et 2 illustrent une première variante de réalisation de l'élément de liaison selon l'invention selon laquelle le support (160, 260) est cylindrique et présente un diamètre extérieur (162, 262) sensiblement identique au diamètre extérieur (151, 251) des spires (152, 252).

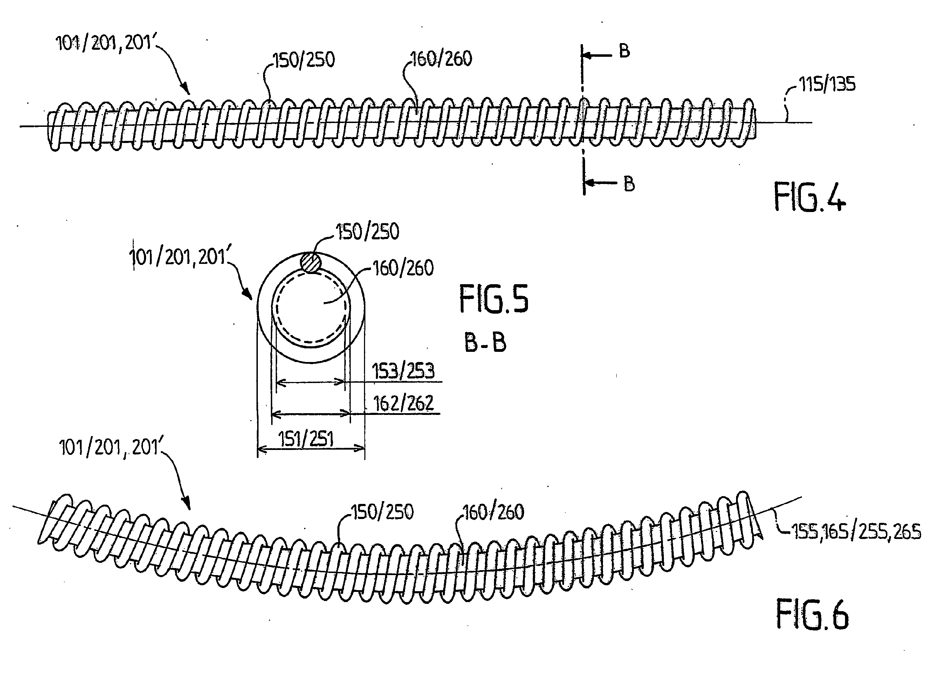

Les figures 4 et 5 illustrent une deuxième variante de réalisation de l'élément de liaison selon l'invention selon laquelle le support (160, 260) est cylindrique et présente un diamètre extérieur (162, 262) inférieur au diamètre extérieur (151, 251) des spires (152, 252), sans toutefois être inférieur au diamètre intérieur (153, 253) des spires (152, 252).

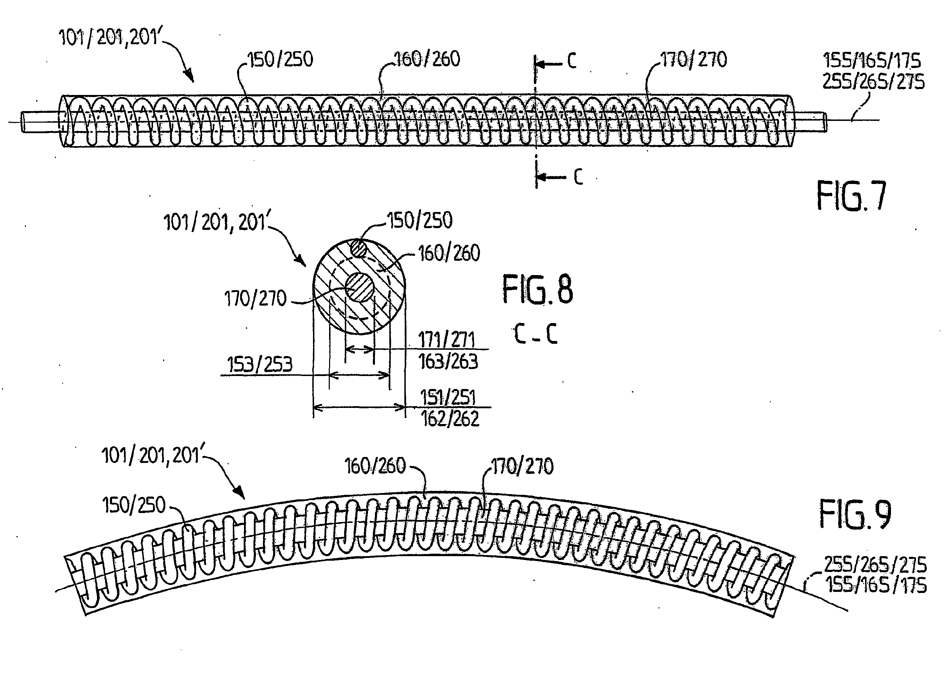

Les figures 7 et 8 illustrent une troisième variante de réalisation de l'élément de liaison selon l'invention selon laquelle le support (160, 260) est tubulaire et présente un diamètre extérieur (162, 262) sensiblement identique au diamètre extérieur (151, 251) des spires (152, 252), et un diamètre intérieur (163, 263) inférieur au diamètre intérieur (153, 253) des spires (152, 252). En outre, l'élément de liaison comporte en son centre une tige (170, 270). Cette tige (170, 270) présente un axe (175, 275) sensiblement coaxial avec l'axe (155, 255) dudit ressort (150, 250).

Cette tige (170, 270) présente en outre un diamètre extérieur (171, 271) inférieur au diamètre intérieur (153, 253) desdites spires (152, 252).

La tige (170, 270) peut être réalisée en métal, ou en alliage métallique, ou encore dans tout type de matériau permettant la réalisation d'une telle tige.

Il est à noter que la tige (170, 270), le ressort hélicoïdal (150, 250), et par voie de conséquence l'élément de liaison (101, 201, 201'), peuvent être cintrés, selon une courbure souhaitée, comme illustré sur les figures 3, 6 et 9, afin de permettre l'adaptation de l'élément de liaison (101, 201, 201') à une configuration particulière.

La structure de l'élément de liaison (101, 201, 201') selon l'invention permet de réaliser la compression et la distraction directement sur les ensembles de connexion implantés, afin de permettre une stabilisation dynamique.

L'élément de liaison dynamique selon l'invention peut également être combiné, dans un même système de fixation, avec des éléments de liaison rigides dont le but est de permettre une ostéosynthèse entre les implants ainsi liés.

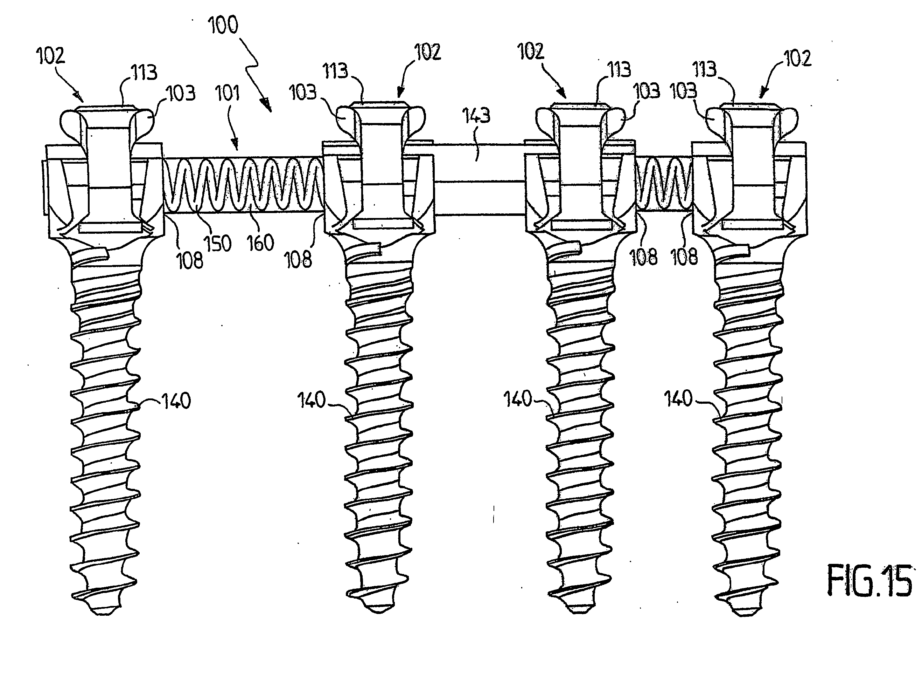

L'élément de liaison (101, 201, 201') selon l'invention peut ainsi également comporter un élément rigidificateur (143, 143'), droit ou cintré, afin de permettre de réaliser une rigidification entre au moins deux ensembles de connexion implantables et permettre ainsi de réaliser une ostéosynthèse entre les implants ainsi liés (figures 15 à 18).

Cet élément rigidificateur (143, 143') est constitué, par exemple, d'une feuille de matériau présentant sensiblement en coupe transversale une forme de U, afin que cette forme puisse être introduite dans les moyens d'accueil des ensembles de connexion et soit fixée par les moyens de fixation de l'élément de liaison sur l'ensemble de connexion implantable.

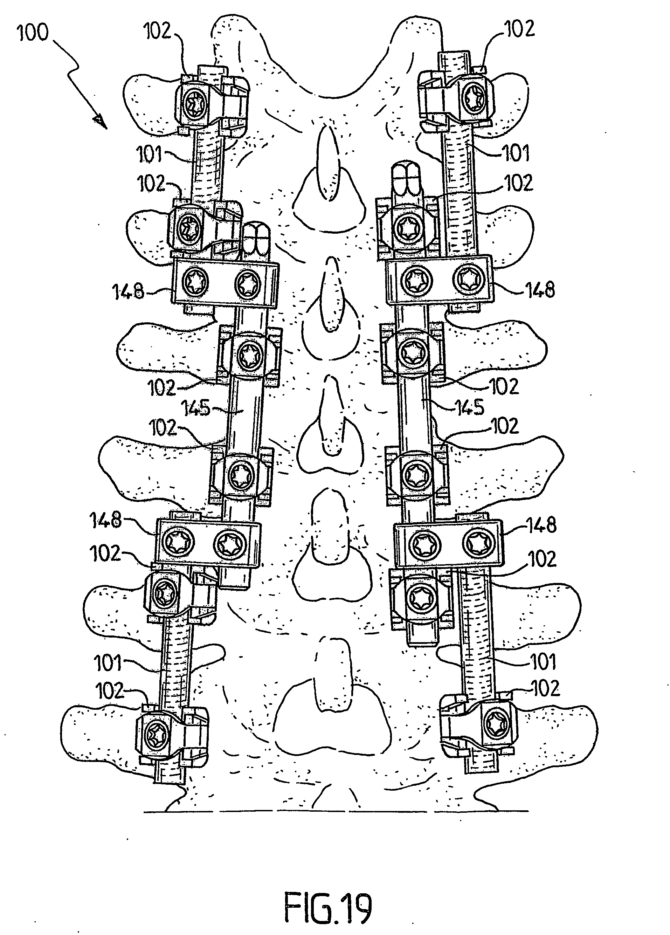

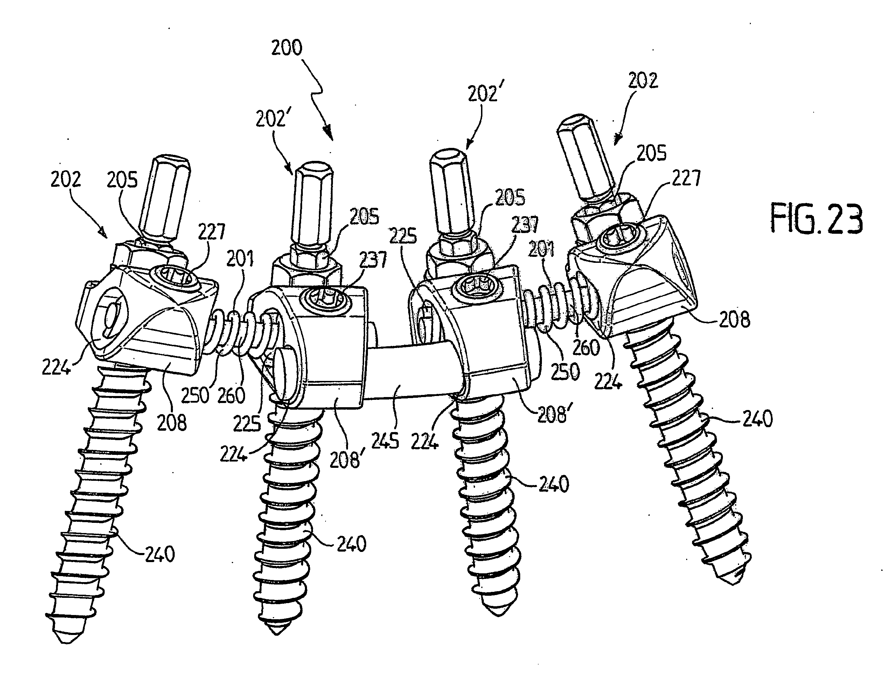

A la place de l'élément rigidificateur (143, 143') associé avec l'élément de liaison (101, 201, 201'), il est possible de positionner un élément de liaison (145, 245), constitué par une tige rigide droite ou cintrée (figures 19 et 23).

Dans les exemples illustrés, le ressort (150, 250) est d'un seul tenant et la forme hélicoïdale est identique sur toute la longueur du ressort.

Toutefois, il est possible d'imaginer que le ressort soit interrompu à l'intérieur de l'élément de liaison, soit par une tige, par exemple parallaxe avec le reste de l'élément de liaison, soit par de la matière constitutive du support (160, 260).

Il est également possible d'imaginer que le pas de la forme hélicoïdale change et ne soit pas constant.

Il peut en outre être imaginé que la forme hélicoïdale ne soit pas circulaire, mais oblongue, que la matière formant le ressort ne présente pas en coupe une forme circulaire, mais oblongue, ou plus simplement que la forme en coupe du support ne soit pas circulaire mais oblongue, afin de procurer plus de résistance sur la grande largeur. Les moyens d'accueil de l'élément de liaison sur l'implant seraient alors modifiés en conséquence.

### Elément de liaison selon l'invention mis en oeuvre à l'aide d'un ensemble de connexion selon la première variante

Selon la première variante, illustrée figures 10 à 18, le système de fixation comporte un élément de liaison (101), un implant (102) avec une pièce de fermeture (103) complémentaire, et une vis de blocage (113), comme montré sur la figure 10.

L'implant (102) présente une tête (105) en forme de fourche, présentant deux bras latéraux (106, 107) délimitant un espace destiné à recevoir l'élément de liaison (101).

Le fond (108) de la fourche présente, en coupe transversale, une forme générale de fer à cheval, avec une courbure concave dans le plan transversal correspond au plan de la figure 10, et une courbure convexe dans le plan complémentaire.

Le rayon de courbure concave correspond sensiblement au rayon extérieur de l'élément de liaison (101). Ce dernier vient ainsi en contact selon une ligne semi-périphérique. Ce contact selon une ligne et non pas selon une surface annulaire autorise un degré de liberté en pivotement, et assure en même temps un blocage plus efficace après serrage que dans le cas d'un simple contact ponctuel.

La pièce de fermeture (103) présente une forme générale de "U", avec deux bras (110, 111) et le fond du U présente un taraudage (112) pour recevoir une vis de blocage (113).

Les bras (110, 111) présentent un écartement permettant la mise en place sur la tête. Les bras (110, 111) présentent à leur extrémité inférieure des épaulements arqués (114, 115) avec une surface supérieure (116, 117) inclinée.

Ces épaulements arqués (114, 115) viennent coopérer avec des moyens de guidage complémentaires (120, 121) prévus sur la tête (105). Ces moyens de guidage présentent également une surface de contact (122, 123) arquées et inclinées, et viennent coopérer avec les surfaces de contact complémentaires (114, 115) lorsque la pièce de fermeture est mise en place sur la tête (105). Elles assurent alors un guidage permettant le pivotement de la pièce de fermeture selon un axe (124) transversal et assurent le verrouillage de la pièce de fermeture (103) sur la tête (105), et donc le blocage de l'élément de liaison (101) après serrage de la vis (113).

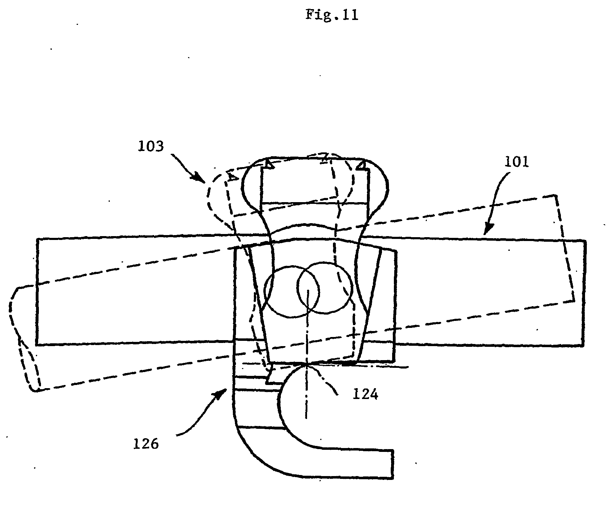

La figure 11 représente une vue de côté qui montre que l'élément de liaison (1) dispose d'un degré de liberté en basculement autour d'un axe transversal (124). Ceci permet de donner une indépendance à l'implant, et de positionner l'implant au moyen d'un crochet (126) sur le pédicule, et indépendamment à chercher la meilleure orientation de l'élément de liaison (1) sans interférence entre ces deux contraintes. La forme de fer à cheval et la mobilité de la pièce de fermeture permet d'adapter le verrouillage et d'éviter la dérotation ou le déplacement de la tige lors du serrage de la vis (113).

Le crochet (126) délimite un espace (127) en "U" pour la liaison avec la lame d'une vertèbre. Afin d'assurer un maintien temporaire, une lame élastique (128) est disposée à l'intérieur de cet espace en "U" et assure un maintien temporaire sur l'os de façon à ce que la lame du crochet ne risque pas de perturber la moelle ou autre structure.

La lame élastique (128) repousse le crochet dans une direction postérieure par rapport au patient, et évite les lésions des tissus nobles pendant la phase de correction par rotation de la tige.

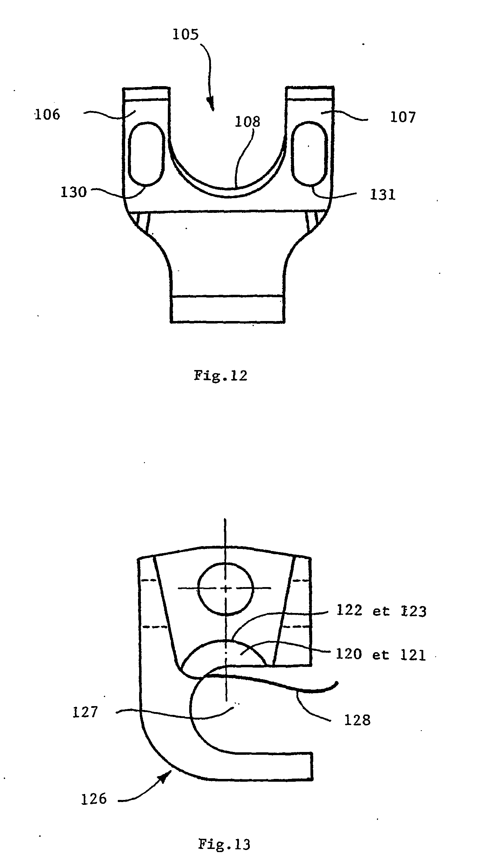

Les figures 12 et 13 représentent des vues de côté de l'implant, sans la pièce de fermeture.

L'implant présente deux échancrures (130, 131) permettant le passage d'un instrument présentant deux becs venant se loger dans les échancrures (130, 131), et un organe venant exercer un effort sur l'élément de liaison (101) pour assurer son déplacement latéral et / ou vertical, en vue de permettre le positionnement de la tige dans la fourche par l'intermédiaire de la pièce de fermeture (103).

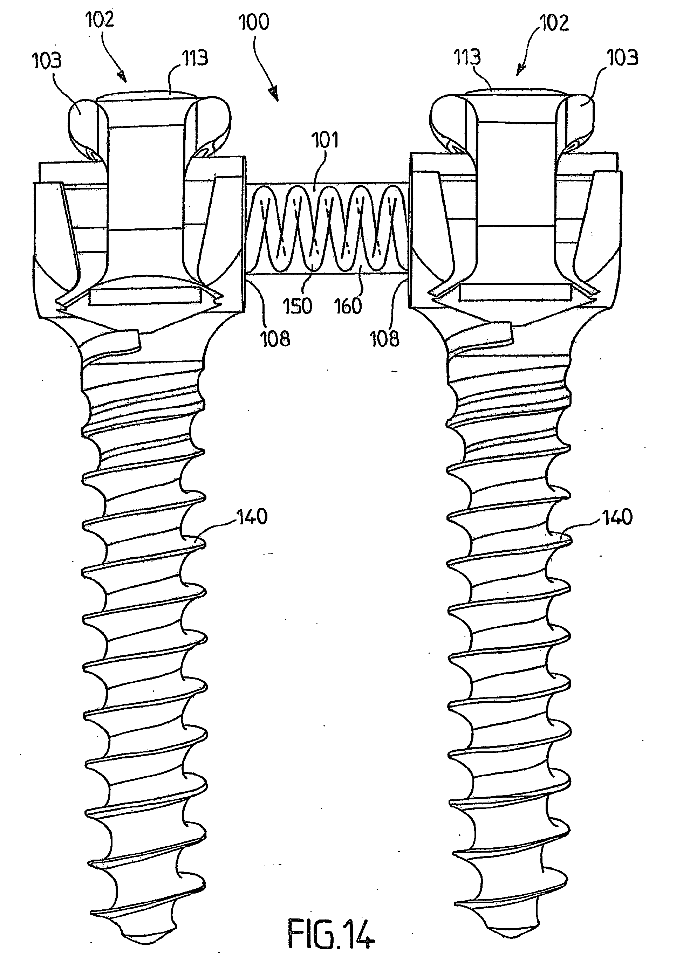

Les figures 11 à 13 illustrent un implant dont les moyens d'ancrage osseux sont constitués d'un crochet (126) ; toutefois, les moyens d'ancrage peuvent également être constitués par une vis osseuse (140), comme illustré sur les figures 14 à 18.

La figure 14 illustre la réalisation d'un système de fixation (100) avec deux implants/ensembles de connexion (102) selon la première variante et un élément de liaison (101) selon l'invention, droit.

L'élément de liaison (101) est disposé dans le fond (108) de chacun des ensembles de connexion (102) et la pièce de fermeture (103) vient refermer les moyens d'accueil de l'élément de liaison. Une vis (113) bloque fermement l'élément de liaison (101) dans la position souhaitée sur chaque ensemble de connexion (102).

Sur la figure 14, l'élément de liaison (101) est constitué d'un ressort (150) noyé dans le support (160) ; toutefois, toutes les variantes de réalisation de l'élément de liaison (101) exposées ci-dessus sont envisageables pour lier les deux ensembles de connexion (102).

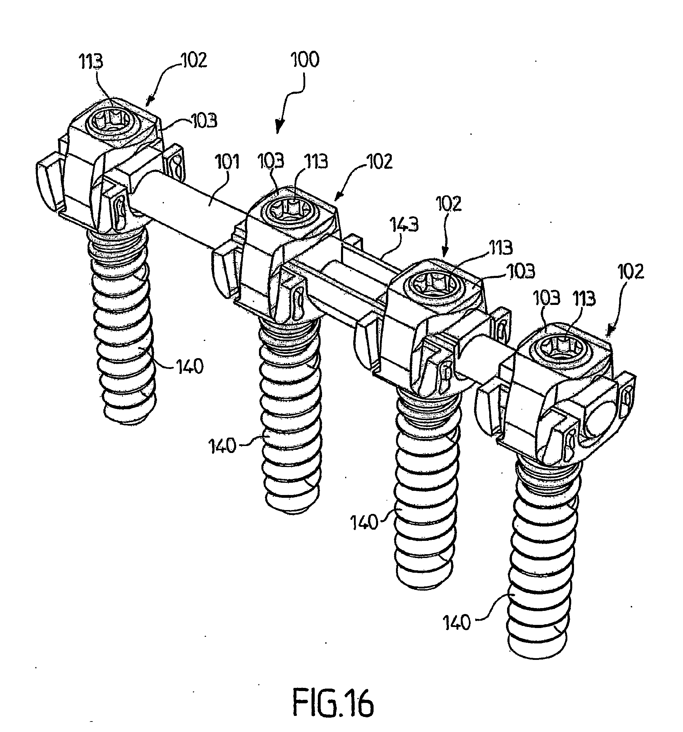

- Les figures 15 et 16 illustrent la réalisation d'un système de fixation (100) avec quatre implants/ensembles de connexion (102) selon la première variante et un élément de liaison (101) selon l'invention unique et droit.

L'élément de liaison (101) est disposé dans le fond (108) de chacun des ensembles de connexion (102) et la pièce de fermeture (103) vient refermer les moyens d'accueil de l'ensemble de connexion. Une vis (113) bloque fermement l'élément de liaison (101) dans la position souhaitée sur chaque ensemble de connexion (102).

Un élément rigidificateur (143) droit est disposé entre les deux ensembles de connexion situés au centre du système de fixation. Cet élément rigidificateur (143) est également disposé dans le fond (108) de chacun des deux ensembles de connexion (102) centraux. La pièce de fermeture (103) vient aussi bloquer l'élément rigidificateur (143) dans les moyens d'accueil de chaque ensemble de connexion central, sous l'élément de liaison (101) et la vis (113) bloque également fermement l'élément rigidificateur (143) dans la position souhaitée sur chaque ensemble de connexion (102) central, par appui sur l'élément de liaison (101) (il n'y a pas de contact entre la vis (113) et l'élément rigidificateur (143)).

Sur la figure 15, l'élément de liaison (101) est constitué d'un ressort (150) noyé dans le support (160), toutefois, toutes les variantes de réalisation de l'élément de liaison (101) exposées ci-dessus sont envisageables pour lier les deux ensembles de connexion (102). Sur la figure 16, la constitution interne de l'élément de liaison (101) n'est pas illustrée afin de ne pas encombrer inutilement le schéma.

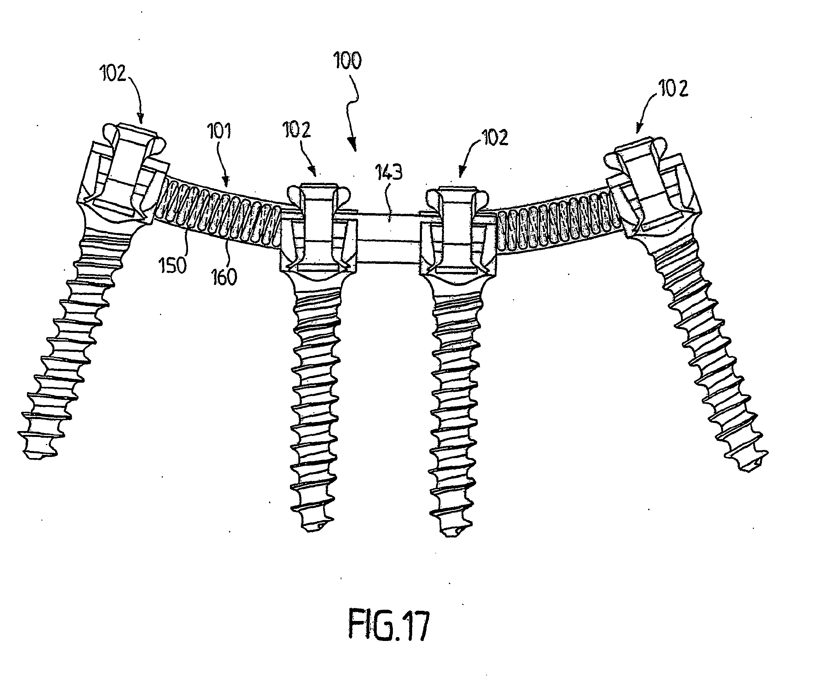

La figure 17 illustre la réalisation d'un système de fixation (100) avec quatre implants/ensembles de connexion (102) selon la première variante et un élément de liaison (101) selon l'invention unique et cintré. Toutefois, dans cette réalisation, l'élément de liaison (101) n'est cintré que d'une part entre le premier et le deuxième ensemble de connexion et d'autre part entre le troisième et le quatrième ensemble de connexion. Un élément rigidificateur (143) droit est disposé entre les deux ensembles de connexion situés au centre du système de fixation.

L'élément de liaison (101) incluant l'élément rigidificateur (143) est disposé dans le fond (108) de chacun des ensembles de connexion (102) et la pièce de fermeture (103) vient refermer les moyens d'accueil de l'ensemble de connexion. Une vis (113) bloque fermement l'élément de liaison (101) dans la position souhaitée sur chaque ensemble de connexion (102).

Sur la figure 17, l'élément de liaison (101) est constitué d'un ressort (150) noyé dans le support (160), toutefois, toutes les variantes de réalisation de l'élément de liaison (101) exposées ci-dessus sont envisageables pour lier les deux ensembles de connexion (102).

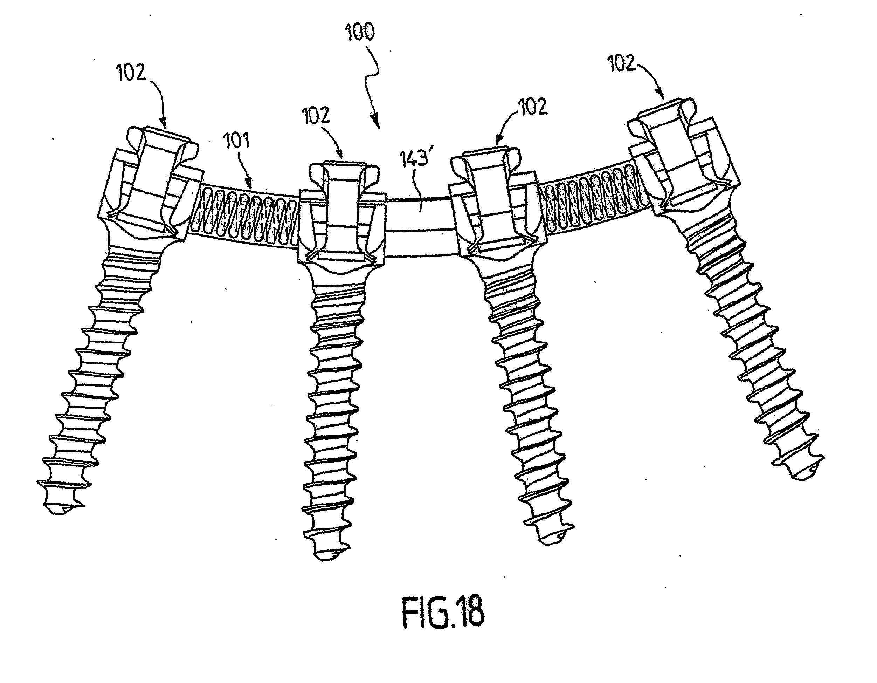

La figure 18 illustre la réalisation d'un système de fixation (100) avec quatre implants/ensembles de connexion (102) selon la première variante et un élément de liaison (101) selon l'invention unique et cintré.

Dans cette réalisation, l'élément de liaison (101) est cintré entre tous les ensembles de connexion (102). Un élément rigidificateur (143') cintré est disposé entre les deux ensembles de connexion situés au centre du système de fixation.

L'élément de liaison (101) incluant l'élément rigidificateur (143') est disposé dans le fond (108) de chacun des ensembles de connexion (102) et la pièce de fermeture (103) vient refermer les moyens d'accueil de l'ensemble de connexion. Une vis (113) bloque fermement l'élément de liaison (101) dans la position souhaitée sur chaque ensemble de connexion (102).

Sur la figure 18, l'élément de liaison (101) est constitué d'un ressort (150) noyé dans le support (160) ; toutefois, toutes les variantes de réalisation de l'élément de liaison (101) exposées ci-dessus sont envisageables pour lier les deux ensembles de connexion (102).

La figure 19 illustre la réalisation d'un système de fixation (100) avec douze implants/ensembles de connexion (102) selon la première variante, implantés chacun dans un pédicule d'une vertèbre. Ces douze implants sont liés entre eux, dans la partie supérieure et inférieure du système de fixation, par quatre éléments de liaison (101) selon l'invention droit et dans la partie centrale par deux éléments de liaison (145) rigides formés par des tiges droites.

La liaison entre les éléments de liaison (145) rigides et les éléments de liaison (101) selon l'invention est opérée à l'aide de quatre moyens de liaison appelés « domino ». Chacun de ces moyens de liaison présente deux moyens d'accueil, pour accueillir un élément de liaison dans chaque moyen d'accueil, et des moyens de retenu constitués par deux écrous venant lier des moyens de fixation auxdits moyens d'accueil.

Comme on peut le constater, du côté gauche, est illustrée une version de l'invention dans laquelle les dominos (148) sont situés à l'extrémité de l'élément de liaison rigide (145), cet élément étant fixé aux pédicules centraux à l'aide de deux implants centraux. Du côté droit, les dominos (148) sont situés à l'extrémité des éléments de liaison (101) selon l'invention, l'élément de liaison rigide (145) étant fixé aux pédicules centraux à l'aide de quatre implants.

Sur la figure 19, les éléments de liaison (101) sont constitués chacun d'un ressort (150) noyé dans le support (160) ; toutefois, toutes les variantes de réalisation de l'élément de liaison (101) exposées ci-dessus sont envisageables pour lier les ensembles de connexion (102) entre eux ou avec les dominos.

### Élément de liaison selon l'invention mis en oeuvre à l'aide d'un ensemble de connexion selon la deuxième variante

Selon la deuxième variante, illustrée figures 20 à 23, le système de fixation comporte un élément de liaison (201), un implant (202, 202') constitué d'un moyen de connexion et d'un moyen d'ancrage, le moyen d'ancrage étant constitué, par exemple, d'une vis osseuse (240) comportant un filetage de type osseux (241), comme montré sur la figure 20.

Le filetage de type osseux (241) présente une fonction principale : celle d'assurer l'ancrage dans l'os. Il peut éventuellement présenter une fonction secondaire : celle de recevoir le système de connexion pour un élément de liaison lorsque la zone de connexion (229) est confondue avec la partie supérieure du filetage osseux (241).

Sur l'extrémité supérieure de la vis (240), un système d'entraînement (205) en rotation est prévu. Ce système par exemple un hexagone, a deux fonctions : d'une part, il permet l'entraînement en rotation de la vis (240) lors de la pénétration dans l'os de celle-ci, et d'autre part, il a également un rôle de blocage en rotation lors du serrage final du mécanisme pour éviter une pénétration plus importante de la vis (240) dans l'os.

Le moyen de connexion permet de réaliser une butée longitudinale le long de la vis (240) par le biais d'un connecteur (208, 208').

Le moyen de connexion peut être constitué par un écrou (219). Dans ce cas, le taraudage intérieur (207) de l'écrou (219) correspond au filetage de la zone de connexion (229) ; c'est-à-dire, dans la version illustrée figures 20 à 23, au filetage osseux (241) de la vis (240). L'écrou (219) est vissé sur la partie non enfouie de l'os de la vis.

L'écrou (219) est dans sa partie inférieure de forme sphérique (220). Cette forme sphérique est destinée à laisser un positionnement libre de l'écrou (219) sur le connecteur (208) où est prévu un logement du même type ou sur l'élément de liaison (201). Cette forme sphérique (220) sert également de butée de positionnement longitudinal avec le connecteur (208) ou avec l'élément de liaison (201). Dans l'application préférentielle, le moyen de connexion est serti dans le connecteur (208) ou dans l'élément de liaison (201) de façon à solidariser les deux pièces tout en laissant une rotation du moyen de connexion sur le connecteur (208) ou sur l'élément de liaison (201).

Un système d'entraînement (221) par exemple un hexagone extérieur est également prévu sur le moyen de connexion au-dessus de sa partie sphérique (220) de façon à permettre son réglage en hauteur ainsi que celui du connecteur (208) ou de l'élément de liaison (201) le long de la vis (240). En conséquence de la possibilité de rotation du connecteur sur le moyen de connexion, un cône (222) d'entrée dans le connecteur (208) est prévu pour le passage de l'ancillaire pour l'entraînement en rotation du moyen de connexion. À titre d'exemple, le débattement angulaire D de l'axe A du connecteur sur le moyen de connexion est dans l'application préférentielle de 30 degrés quand l'ancillaire d'entraînement en rotation du moyen de connexion est en place, comme illustré figure 20.

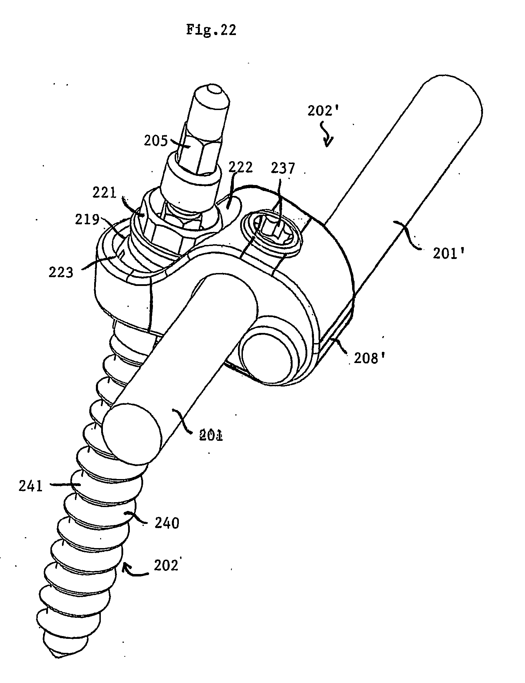

Des fentes (223), visibles figure 22, sont usinées dans la partie sphérique du moyen de connexion de façon à créer une déformation lors du serrage définitif du système. Cette déformation a pour but de bloquer en rotation la vis (226).

Les fentes (223) peuvent être positionnées transversalement ou longitudinalement.

Les fentes longitudinales sont de préférence débouchantes dans la partie inférieure de la forme sphérique (220). Elles peuvent être au nombre de une, deux, trois, quatre, cinq, ou plus.

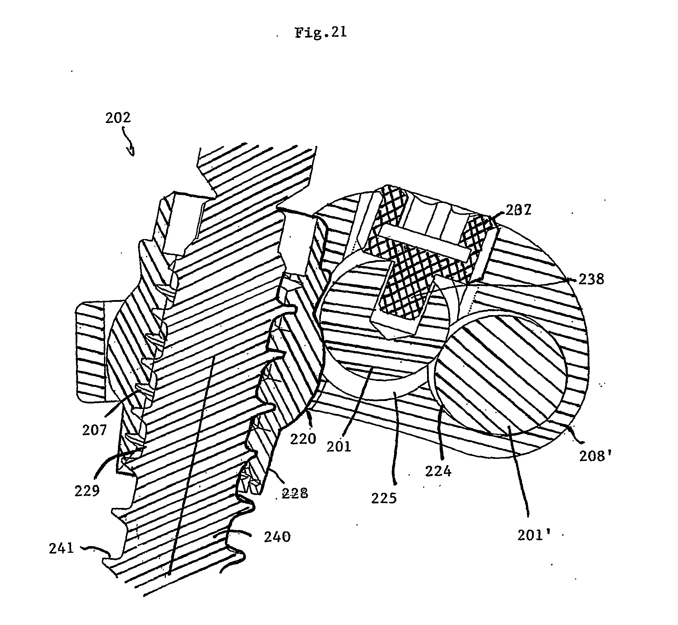

Le moyen de connexion comporte dans sa partie inférieure une jupe (228) visible figure 21. Cette jupe (228) permet d'avoir une transition mécanique entre la vis (240) et la forme sphérique (220). En effet, une transition trop franche favoriserait une rupture de la vis à la base de la forme sphérique (220) lors d'efforts dynamiques. La jupe (228) permet ainsi de mieux répartir la contrainte d'encastrement de la vis dans l'écrou.

Cette jupe (228) peut être filetée sur son extrémité pour faciliter sa pénétration dans l'os.

La zone de connexion (229) de la vis (240) peut être ménagée dans la partie supérieure du filetage osseux et présenter un filetage identique au filetage osseux, comme illustré, mais elle peut également présenter un filetage différent, ou encore ne pas être filetée.

Dans ce dernier cas, le moyen de connexion présente par conséquent une paroi intérieure lisse et ne constitue pas un écrou.

Dans la version de base de la deuxième variante, illustrée figure 20, le connecteur (208) est pourvu d'un seul emplacement (224) pour recevoir un élément de liaison (201). Cet emplacement (224) peut être en forme de oblong dans le cas d'un connecteur (208) de type fermé, soit en forme de « U » débouchante sur une des faces dans le cas d'un connecteur de type ouvert. Dans le cas d'un connecteur (208) de type fermé, l'élément de liaison (201) doit être enfilée dans celui-ci, tandis que dans le cas d'un connecteur ouvert, l'élément de liaison (201) peut être introduite sur le connecteur postérieurement ou latéralement.

L'emplacement (224) de réception de l'élément de liaison (201) est prévu de manière à ce que l'élément de liaison (201) puisse venir en appui sur la forme sphérique (220) de l'écrou (219), comme illustré figure 20.

Il est donc entendu que l'élément de liaison (201) par rapport à la vis (226) est libre, avant fixation définitive, dans trois axes de rotations et dans deux directions de translations :
- Rotation du connecteur (208) et donc de l'élément de liaison (201) autour du moyen de connexion dans deux axes perpendiculaires à la vis (240) ;
- Rotation du connecteur (208) et donc de l'élément de liaison (201) autour du moyen de connexion dans un axe identique à celui de la vis (240) ;
- Translation de l'élément de liaison (201) dans le connecteur (208) suivant l'axe de celle-ci ;
- Translation du connecteur (208) et donc de l'élément de liaison (201) le long de la vis (240) grâce à la possibilité de réglage du moyen de connexion.

En outre, la rotation de l'élément de liaison (201) sur lui-même peut offrir un degré de liberté supplémentaire à l'ensemble de connexion (202).

Le connecteur (208) possède également un système de blocage. Dans la version de base illustrée figure 20, ce système de blocage est un écrou (226) pourvu d'un système d'entraînement (227) pour appliquer un couple suffisant à la bonne tenue mécanique de l'ensemble.

Le blocage d'une telle connexion est assuré par la pression de l'élément de liaison (201) sur la forme sphérique (220). L'élément de liaison (201) étant solidaire du connecteur (208) par la pression opérée par l'écrou (226), les degrés de liberté sont alors tous figés.

Dans une version plus complexe de la deuxième variante, illustrée figures 21 et 22, l'ensemble de connexion (202') peut accueillir deux éléments de liaison (201, 201').

Le connecteur (208') comporte un emplacement de blocage (225), dans lequel peut être introduit un élément de liaison (201), l'autre élément de liaison (201') étant introduit dans l'emplacement (224).

L'emplacement de blocage (225) débouche dans une direction sensiblement perpendiculaire pour l'introduction d'un bouchon de blocage (237), comme illustré figure 20.

Dans cette version, le serrage de l'ensemble est assuré par la force qui est opérée à la fois sur l'élément de liaison (201') et sur la partie sphérique (220) par l'élément de liaison (201).

Il est nécessaire de prévoir une pluralité de connecteurs, afin de pouvoir choisir celui dont la distance entre la vis et l'élément de liaison ou les éléments de liaison est adéquat.

La figure 23 illustre la réalisation d'un système de fixation (200) avec deux implants/ensembles de connexion (202) selon la version de base de la deuxième variante aux extrémités et deux implants/ensembles de connexion (202') selon la version plus complexe de la deuxième variante au centre.

Un élément de liaison (201) selon l'invention, cintré, est disposé entre le premier et le deuxième ensemble de connexion et un autre élément de liaison (201) selon l'invention, cintré, est disposé entre le troisième et le quatrième ensemble de connexion. Un élément de liaison (245) cintré et rigide est disposé entre les deux ensembles de connexion situés au centre du système de fixation.

Dans les ensembles de connexion (202), les éléments de liaison (201) sont disposés, respectivement, dans les emplacements (224) des connecteurs (208).

Dans les ensembles de connexion (202'), les éléments de liaison (201) sont disposés, respectivement, dans les emplacements de blocage (225) des connecteurs (208') et l'élément de liaison (245) est disposé, respectivement, dans les emplacements (224) des connecteurs (208').

Sur la figure 23, les éléments de liaison (201) cintrés sont constitués, chacun d'un ressort (250) et d'un support (260), du type de celui illustré figure 6 ; toutefois, toutes les variantes de réalisation de l'élément de liaison (201) exposées ci-dessus sont envisageables pour lier les ensembles de connexion (202, 202').

L'invention est décrite dans ce qui précède à titre d'exemple non limitatif. Il est entendu que l'homme du métier peut réaliser diverses variantes, notamment en remplaçant le crochet par une vis pédiculaire, ou une vis vertébrale pour la pose sur la face antéro-latérale du rachis.

## Revendications

1. Elément de liaison (101, 201, 201') pour un système de fixation rachidien (100, 200), destiné à lier au moins deux ensembles de connexion implantables (102, 202, 202'), constitué, au moins partiellement, d'un support (160, 260) en matériau polymère et de deux tiges, une première tige (170, 270), cintrée ou non, sensiblement coaxiale avec ledit support (160, 260), et une seconde tige formée de spires (152, 252) entourant ladite première tige (170, 270), lesdites spires (152, 252) étant, au moins partiellement, noyées dans ledit support (160, 260).

2. Elément de liaison (101, 201, 201') selon la revendication 1, **caractérisé en ce que** ledit support (160, 260) est constitué d'une forme sensiblement tubulaire ou cylindrique.

3. Elément de liaison (101, 201, 201') selon la revendication 1 ou la revendication 2, **caractérisé en ce que** les spires (152, 252) de la seconde tige forment un ressort hélicoïdal (150, 250) présentant un axe sensiblement parallèle avec l'axe (165, 265) dudit support (160, 260).

4. Elément de liaison (101, 201, 201') selon la revendication 3, **caractérisé en ce que** ladite première tige (170, 270) est sensiblement coaxiale avec ledit ressort (150, 250).

5. Elément de liaison (101, 201, 201') selon la revendication 3 ou la revendication 4, **caractérisé en ce que** ladite première tige (170, 270) présente un diamètre extérieur (171, 271) inférieur au diamètre intérieur (153, 253) desdites spires (152, 252).

6. Elément de liaison (101, 201, 201') selon l'une quelconque des revendications 1 à 5, **caractérisé en ce qu'**il comporte un élément rigidificateur (143, 143'), droit ou cintré, entourant partiellement le support (160, 260), de sorte à rigidifier sur une portion ledit élément de liaison (101, 201, 201').

7. Elément de liaison (101, 201, 201') selon la revendication 6, **caractérisé en ce que** ledit élément rigidificateur (143, 143') est constitué d'une feuille de matériau présentant sensiblement en coupe transversale une forme de U.

8. Système de fixation rachidien (100, 200) comportant au moins deux ensembles de connexion implantables (102, 202, 202') liés à l'aide d'au moins un élément de liaison (101, 201, 201') selon l'une quelconque des revendications 1 à 7.

9. Système de fixation rachidien (100, 200) selon la revendication 8, comportant un élément de liaison selon la revendication 6, **caractérisé en ce que** ledit élément rigidificateur (143, 143') est fixé au moins aux deux ensembles de connexion implantables (102, 202, 202').

10. Système de fixation rachidien (100, 200) selon la revendication 8 ou la revendication 9, **caractérisé en ce qu'**il comporte en outre au moins un élément de liaison rigide (145, 245) fixé à au moins deux ensembles de connexion implantables et relié à au moins un élément de liaison (101, 201, 201') par un moyen de liaison.

## Claims

1. Linking element (101, 201, 201') for a spinal fixing system (100, 200), intended to link at least two implantable connection assemblies (102, 202, 202'), comprised at least partially of a support (160, 260) in polymer material and two rods, a first rod (170, 270) either bent or not, roughly coaxial with the said support (160, 260) and a second rod formed of turns (152, 252) surrounding the said first rod (170, 270), the said turns of which (152, 252) being at least partially buried in the said support (160, 260).

2. Linking element (101, 201, 201') according to claim 1, **characterised in that** the said support (160, 260) is comprised of a roughly tubular or cylindrical shape.

3. Linking element (101, 201, 201') according to claim 1 or claim 2, **characterised in that** the turns (152, 252) of the second rod form a helical spring (150, 250) presenting an axis roughly parallel to the axis (165, 265) of the said support (160, 260).

4. Linking element (101, 201, 201') according to claim 3, **characterised in that** the said rod (170, 270) is roughly coaxial with the said spring (150, 250).

5. Linking element (101, 201, 201') according to claim 3 or claim 4, **characterised by** the fact that the said first rod (170, 270) has an external diameter (171, 271) less than the internal diameter (153, 253) of the said springs (152, 252).

6. Linking element (101, 201, 201') according to any of claims 1 to 5, **characterised in that** it comprises a stiffening element (143, 143'), straight or bent, partially surrounding the support (160, 260) in such a way as to stiffen the said linking element (101, 201, 201') over a section.

7. Linking element (101, 201, 201') according to claim 6, **characterised in that** the said stiffening element (143, 143') is constituted of a sheet of material with roughly a U shape in the transversal cross-section.

8. Spinal fixing system (100, 200) comprising at least two implantable connection assemblies (102, 202, 202') linked by means of at last one linking element (101, 201, 201') according to any of claims 1 to 7.

9. Spinal fixing system (100, 200) according to claim 8, comprising a linking element according to claim 6, **characterised in that** the said stiffening element (143, 143') is fixed to at least two implantable connection assemblies (102, 202, 202').

10. Spinal fixing system (100, 200) according to claim 8 or claim 9, **characterised in that** it comprises in addition at least one rigid linking element (145, 245) fixed to at least two implantable connection assemblies and linked to at least one linking element (101, 201, 201') by a means of linkage.

## Patentansprüche

1. Verbindungselement (101, 201, 201') für ein Befestigungssystem an der Wirbelsäule (100, 200), das zur Verbindung von wenigstens zwei implantierbaren Anschlusselementen (102, 202, 202') bestimmt ist und wenigstens teilweise aus einem Träger (160, 260) aus Polymermaterial und zwei Stiften, einem ersten (170, 270), gebogenen oder nicht gebogenen, mit dem genannten Träger (160, 260) deutlich koaxialen Stift und einem zweiten, aus den genannten ersten Stift (170, 270) umgebenden Windungen (152, 252) geformten Stift gebildet wird, wobei die genannten Windungen (152, 252) wenigstens teilweise in dem genannten Träger (160, 260) versenkt sind.

2. Verbindungselement (101, 201, 201') gemäß Anspruch 1, **dadurch gekennzeichnet, dass** der genannte Träger (160, 260) aus einer deutlich röhrenförmigen oder zylindrischen Form gebildet wird.

3. Verbindungselement (101, 201, 201') gemäß Anspruch 1 oder Anspruch 2, **dadurch gekennzeichnet, dass** die Windungen (152, 252) des zweiten Stiftes eine eine zur Achse (165, 265) des genannten Trägers (160, 260) deutlich parallele Achse aufweisende zylindrische Schraubenfeder (150, 250) bilden.

4. Verbindungselement (101, 201, 201') gemäß Anspruch 3, **dadurch gekennzeichnet, dass** der genannte erste Stift (170, 270) deutlich koaxial zu der genannten Feder (150, 250) ist.

5. Verbindungselement (101, 201, 201') gemäß Anspruch 3 oder Anspruch 4, **dadurch gekennzeichnet, dass** der genannte erste Stift (170, 270) einen kleineren Außendurchmesser (171, 271) als den Innendurchmesser (153, 253) der genannten Windungen (152, 252) aufweist.

6. Verbindungselement (101, 201, 201') gemäß Anspruch 1 bis 5, **dadurch gekennzeichnet, dass** es ein gerades oder gebogenes, den Träger (160, 260) teilweise umfassendes Versteifungselement (143, 143') umfasst, so dass das genannte Verbindungselement (101, 201, 201') auf einem Abschnitt versteift wird.

7. Verbindungselement (101, 201, 201') gemäß Anspruch 6, **dadurch gekennzeichnet, dass** das genannte Versteifungselement (143, 143') aus einem deutlich einen U-förmigen Querschnitt aufweisenden Materialblatt gebildet wird.

8. Befestigungssystem an der Wirbelsäule (100, 200) mit wenigstens zwei mithilfe wenigstens eines Verbindungselements (101, 201, 201') gemäß Anspruch 1 bis 7 verbundenen implantierbaren Anschlussstrukturen (102, 202, 202').

9. Befestigungssystem an der Wirbelsäule (100, 200) gemäß Anspruch 8 mit einem Verbindungselement gemäß Anspruch 6, **dadurch gekennzeichnet, dass** das genannte Versteifungselement (143, 143') wenigstens an den beiden implantierbaren Anschlussstrukturen (102, 202, 202') befestigt ist.

10. Befestigungssystem an der Wirbelsäule (100, 200) gemäß Anspruch 8 oder Anspruch 9, **dadurch gekennzeichnet, dass** es darüber hinaus wenigstens ein an wenigstens zwei implantierbaren Anschlussstrukturen befestigten und mit wenigstens einem Verbindungselement (101, 201, 201') durch ein Verbindungsmittel verbundenes steifes Verbindungselement (145, 245) umfasst.
